# EUROPEAN PATENT SPECIFICATION

(11) **EP 1 654 222 B1**
(45) Date of publication and mention of the grant of the patent: **18.09.2013**
(21) Application number: 04748172.6
(22) Date of filing: 27.07.2004
(51) Int. Cl.: C07C 263/18, C07C 265/04

(54) **Stabilized (meth)acryloyloxyalkyl isocyanate, a process for stabilization thereof and a process for preparation of the same**
Stabilisiertes (Meth)Acryloyloxyisocyanat, Verfahren zu seiner Stabilisierung und seiner Herstellung
Isocyanate de (méth)acryloyloxyalkyle stabilisé, son procédé de stabilisation et son procédé de préparation

(30) Priority: 31.07.2003 JP 2003283694
(43) Date of publication of application: 10.05.2006
(73) Proprietor: Showa Denko K.K., Tokyo 105-8518 (JP)
(72) Inventor: MORINAKA, K., SHOWA DENKO K.K., 111, Aza-Nagayachi, Kawanuma-gun, Fukushima 9693431 (JP); ISHIKAWA, T., SHOWA DENKO K.K., 111, Aza-Nagayachi, Kawanuma-gun, Fukushima 9693431 (JP); HOSHI, K., SHOWA DENKO K.K., 111, Aza-Nagayachi, Kawanuma-gun, Fukushima 9693431 (JP)
(74) Representative: Strehl Schübel-Hopf & Partner
(86) International application number: PCT/JP2004/011017
(87) International publication number: WO 2005/012236

(56) References cited:
- EP-A- 0 645 372
- EP-A- 0 936 214
- DE-A- 3 225 247
- US-A- 2 821 544
- US-A- 3 247 236
- DATABASE WPI Section Ch, Week 198408 Derwent Publications Ltd., London, GB; Class A41, AN 1982-61474E XP002302504 & JP 59 007147 A (DOW CHEM CO) 14 January 1984 (1984-01-14)
- DATABASE WPI Section Ch, Week 199028 Derwent Publications Ltd., London, GB; Class A41, AN 1990-214434 XP002302505 & JP 02 145555 A (SHOWA RHODIA KAGAKU) 5 June 1990 (1990-06-05)

## Description

### TECHNICAL FIELD

The present invention relates to a stabilized (meth)acryloyloxyalkyl isocyanate and a process for stabilization thereof. More particularly, it relates to a stabilized (meth)acryloyloxyalkyl isocyanate having a small hydrolyzable chlorine content and good storage stability and a process for stabilizing a (meth)acryloyloxyalkyl isocyanate prepared by using phosgene which process comprises decreasing the amount of a hydrolyzable chlorine with purification.

In the present specification, "(meth)acryloyl" means acryloyl or methacryloyl. Further, "(meth)acryloyloxyalkyl isocyanate" means a composition substantially consisting of (meth)acryloyloxyalkyl isocyanate capable of containing a slight amount of an acidic gas and a hydrolyzable chlorine, except for the particular comment on it as a compound.

### BACKGROUND ART

(Meth)acryloyloxyalkyl isocyanates typified by methacryloyloxyethyl isocyanate are compounds containing, per molecule, an isocyanate group having high reactivity with a compound having an active hydrogen, such as compounds having a substituent e.g. a hydroxyl group, or primary or secondary amino group, and a carbon-carbon double bond capable of vinyl polymerization and are industrially very useful compounds so that they are used in various uses, for example, paints and coating materials, adhesives, photo-resists, dental materials and magnetic recording materials.

As described in US Patent No.2,821,544 and JP-AS54(1979)-5921, this compound is prepared by using a phosgene and generally contains impurities called as hydrolyzable chlorine.

When urethane acrylates are prepared using a (meth)acryloyloxyalkyl isocyanate containing hydrolyzable chlorine, the hydrolyzable chlorine works as a catalyst poison and also a chlorine compound contaminated during the preparation affects weathering resistance and corrosion resistance. Particularly, when the (meth)acryloyloxyalkyl isocyanate containing hydrolyzable chlorine is used to photo-resist materials for electronic apparatus parts, the presence of hydrolyzable chlorine may induce a serious problem.

Conventionally, various methods of decreasing the amount of hydrolyzable chlorine in an isocyanate compound are proposed. For example, JP-A-H11(1999)-228523 discloses a method of preparing a (meth)acryloyloxyalkyl isocyanate containing a very small amount of hydrolyzable chlorine by adding an amine and/or a compound containing an imidazole and an epoxy group, heating, followed by distillation.

However, the present inventors examined on the method and found that the (meth)acryloyloxyalkyl isocyanate prepared by the above method has low storage stability and generates insoluble components and becomes turbid and thereby cannot withstand subsequent use. On this account, the present inventors studied to improve the storage stability of this (meth)acryloyloxyalkyl isocyanate.

As a method of improving the stability of the isocyanate compound, JP-A-H7(1995)-149705 discloses that a polyisocyanate prepared by a non-phosgene method is unstable because the isocyanate group reacts to generate an oligomer with the elapse of time, and the polyisocyanate is stabilized by inclusion of carbon dioxide. However, it does not examine at all on an isocyanate prepared by a phosgene method, particularly on (meth)acryloyloxyalkyl isocyanates.

US Patent No.3,247,236 discloses that a diisocyanate prepared by a phosgene method is stabilized by carbon dioxide or sulfur dioxide. However, in the examples, only isocyanates containing a relatively large amount of hydrolyzable chlorine, namely,ones having a hydrolyzable chlorine content of not less than 60 ppm are used for the test.

DE 32 25 247 A, DATABASE WPI, AN 1982-61474E; JP 59 007147 A and DATABASE WPI, AN 1990-214434; JP 02 145555 A disclose stabilized (meth)acryloyloxyalkyl isocyanate obtained by using nitrogen oxide or sulfur dioxide gas during distillation.

### DISCLOSURE OF INVENTION

Such a phenomenon that when a (meth)acryloyloxyalkyl isocyanate is prepared using phosgene and purified to decrease the amount of hydrolyzable chlorine contained therein, the resulting (meth)acryloyloxyalkyl isocyanate is unstable and shows whitely turbid has not been reported previously and further a method for improving the stability is not known.

The present invention is intended to solve the problems associated with the prior art as described above. It is an object of the invention to provide a stabilized (meth)acryloyloxyalkyl isocyanate, a process for stabilization thereof and a process for preparation of the same. More particularly, the invention provides a (meth)acryloyloxyalkyl isocyanate having a small hydrolyzable chlorine content and good storage stability and a process for stabilizing a (meth)acryloyloxyalkyl isocyanate prepared using phosgene which process comprises decreasing the amount of a hydrolyzable chlorine with purification.

The summary of the present invention is as follows.
[1] A (meth)acryloyloxyalkyl isocyanate having a hydrolyzable chlorine content of not more than 30 ppm based on the (meth)acryloyloxyalkyl isocyanate and containing dissolved carbon dioxide in an amount of not less than 20 ppm based on the (meth)acryloyloxyalkyl isocyanate.
[2] The (meth)acryloyloxyalkyl isocyanate according to [1] which is prepared by using phosgene.
[3] The (meth)acryloyloxyalkyl isocyanate according to [1] or [2] wherein the (meth)acryloyloxyalkyl isocyanate is (meth)acryloyloxyethyl isocyanate.
[4] A process for stabilizing a (meth)acryloyloxyalkyl isocyanate, which process comprises dissolving carbon dioxide in the (meth)acryloyloxyalkyl isocyanate in an amount of 20 to 250 ppm based on the (meth)acryloyloxyalkyl isocyanate with forced blowing,
   wherein the (meth)acryloyloxyalkyl isocyanate is a high purity (meth)acryloyloxyalkyl isocyanate which is prepared by decreasing the amount of hydrolyzable chlorine with purification and has a hydrolyzable chlorine content of not more than 30 ppm based on the (meth)acryloyloxyalkyl isocyanate.
[5] The process for stabilizing a (meth)acryloyloxyalkyl isocyanate according to [4], wherein the (meth)acryloyloxyalkyl isocyanate is prepared by using phosgene.
[6] The process for stabilizing a (meth)acryloyloxyalkyl isocyanate according to [4] or [5] wherein the (meth)acryloyloxyalkyl isocyanate is (meth)acryloyloxyethyl isocyanate.

According to the present invention, provided is a stable (meth)acryloyloxyalkyl isocyanate, and particularly a (meth)acryloyloxyalkyl isocyanate having a small content of hydrolyzable chlorine and excellent storage stability.

According to the process for stabilization, it is possible to stabilize a (meth)acryloyloxyalkyl isocyanate, and particularly to sufficiently stabilize a (meth)acryloyloxyalkyl isocyanate prepared by decreasing the amount of hydrolyzable chlorine with purification.

### BEST MODE FOR CARRYING OUT THE INVENTION

The present invention is described in detail below.

The (meth)acryloyloxyalkyl isocyanate used in the invention is a compound represented by the following formula (I).

CH₂ = C(R¹) - COO - R² - NCO (I)

In the formula (I), R¹ shows hydrogen or a methyl group and R² shows an alkylene group, preferably an alkylene group having 2 to 6 carbon atoms.

In the present invention, particularly, it is preferred to use the compound of the formula (I) wherein R² is an ethylene group, that is, (meth)acryloyloxyethyl isocyanate, especially methacryloyloxyethyl isocyanate. The (meth)acryloyloxyethyl isocyanate has high reactivity, easy acquisition and easy handling among the compounds of the formula (I).

The present inventors have been studied on decreasing hydrolyzable chlorine in order to use (meth)acryloyloxyalkyl isocyanates for electronic materials. Particularly, in the case of preparing isocyanates using phosgene, hydrolyzable chlorine is always contained and it is not easy to decrease the hydrolyzable chlorine.

In order to decrease the hydrolyzable chlorine, the present inventors have studied on a process for treating with an epoxy compound and distilling off and a process for treating with an epoxy compound and an amine and distilling off. In these processes, when purification is advanced and then the amount of the hydrolyzable chlorine is not more than 30 ppm, particularly not more than 10 ppm, it is found that the stability of (meth)acryloyloxyalkyl isocyanate rapidly lowers and thereby a white turbidity is induced by about 1 day.

Therefore, the present inventors studied on a process for stabilizing this unstable (meth)acryloyloxyalkyl isocyanate and found that the storage stability of this (meth)acryloyloxyalkyl isocyanate is markedly improved by dissolving carbon dioxide in the (meth)acryloyloxyalkyl isocyanate with forced blowing.

Among the (meth)acryloyloxyalkyl isocyanates, methacryloyloxyethyl isocyanate has such a phenomenon that the reactivity of isocyanate is changed by the presence of hydrogen chloride. But, the reactivity thereof is hardly changed by the presence of carbon dioxide, and further the presence of carbon dioxide hardly influences the reactivity (stability) of double bonding, rather, the stability is enhanced to some extent.

Respecting the amount of dissolved carbon dioxide, the amount that the acidic gas is naturally dissolved is insufficient. For example, it is necessary to forcedly dissolve the acidic gas in the (meth)acryloyloxyalkyl isocyanate by blowing the gas by means of a tube or to dissolve carbon dioxide by adding a dry ice.

From the viewpoint of obtaining sufficient storage stability, it is necessary to dissolve the acidic gas in a concentration of not less than 20 ppm based on the (meth)acryloyloxyalkyl isocyanate. The upper limit of the acidic gas concentration is up to the amount of the saturation (optionally up to the amount of somewhat supersaturation) and the upper limit of the carbon dioxide concentration is about 250 ppm.

The amount of the acidic gas dissolved in the (meth)acryloyloxyalkyl isocyanate is regulated by, for example, regulating the blown amount thereof, or by blowing the acidic gas sufficiently and then degassing with vacuum.

In the case of distilling through the above described purification step, the distillation is usually carried out in vacuo in many cases, but it may be carried out with feeding carbon dioxide into a system.

In the present specification, the concentration of carbon dioxide dissolved (ppm: value of mass/mass) is measured by a gas chromatography mass analyzer. The measuring method is as follows.

### (1) Calibration

- At least two specimens having a different concentration of dissolved carbon dioxide each other are prepared by blowing carbon dioxide to an organic solvent such as toluene. With regard to each of these specimens, the concentration of carbon dioxide dissolved is measured by other means, for example, a total organic carbon analyzer.
- Each of the above specimens is measured by a gas chromatography mass analyzer, and a calibration curve is determined from the area value of the resulting carbon dioxide and the concentration of carbon dioxide dissolved measured by the other means.

### (2) Measurement of Specimen

- A specimen is measured by a gas chromatography mass analyzer and the concentration of carbon dioxide dissolved is calculated from the area value of the resulting carbon dioxide and the above calibration curve.

The (meth)acryloyloxyalkyl isocyanate used in the process of the present invention is prepared by, for example, producing using a phosgene and decreasing the content of hydrolyzable chlorine with purification. Examples of the purification process may include a process of treating by adding an epoxy group containing compound to (meth)acryloyloxyalkyl isocyanate and a process of treating by adding an epoxy group containing group, an amine and/or an imidazole and thereafter distilling. These purification processes are known in detail as prior arts disclosed in for example, JP-A-9(1997)-323968 and JP-A-11(1999)-228523.

In the (meth)acryloyloxyalkyl isocyanate in which the amount of hydrolyzable chlorine is decreased, thus prepared, when the content of hydrolyzable chlorine is less than 30 ppm, an unstable state such as a white turbidity is caused with time. Further, when the content is less than 10 ppm, an unstable state is rapidly caused.

However, as described above, forced dissolution of the acidic gas in a concentration of not less than 20 ppm in this unstable (meth)acrylolyloxyalkyl isocyanate prevents it from becoming whitely turbid and gives sufficient storage stability to it.

The (meth)acryloyloxyalkyl isocyanate thus prepared has a small content of hydrolyzable chlorine and sufficient storage stability so that it can be suitably used to electronic material uses in particular.

In the present specification, the amount of hydrolyzable chlorine is a value of chlorine determined by a analysis method described in Article 5.7, JIS K 1556 (tolylene diisocyanate testing method) or an analysis method similar to the above method in principal.

In Examples described later, the content of hydrolyzable chlorine was analyzed by introducing 35 ml of methyl alcohol, 15 ml of water and 5 g of a specimen into a 100 ml volume Erlenmeyer flask, fixing a reflux condenser to the flask and refluxing with heat for 30 min, thereafter cooling to room temperature and conducting potentiometric titration by the use of a N/100 silver nitrate solution.

The chlorine compound containing hydrolyzable chlorine (in which hydrolyzable chlorine is bonded) determined by the above method is presumed to be not a specific compound but a chlorine compound of plural kinds. It is considered that chlorine in a mixture state composed of plural kinds of chlorine compounds. Specifically, in the case of representing isocyanate alkyl(meth)acrylate by R-NCO, it is considered that examples thereof may include compounds represented by R-NH-COCl, R-NCl₂, R-N=C(Cl)-R'·HCl (R' is a vinyl group or isopropenyl group), but the details thereof are unclear. Furthermore, it is considered that even in the case of forcedly dissolving hydrogen chloride, (meth)acryloyloxyalkyl isocyanate is stabilized, but the amount of hydrolyzable chlorine is increased.

### [Example]

Hereinafter, the present invention is described in more detail with reference to the examples.

In Examples and Comparative Examples, the content of hydrolyzable chlorine was determined by introducing 35 ml of methyl alcohol, 15 ml of water and 5 g of a specimen into a 100 ml volume Erlenmeyer flask, fixing a reflux condenser to the flask and refluxing with heat for 30 min, thereafter cooling to room temperature and conducting potentiometric titration by the use of a N/100 silver nitrate solution.

Further, the concentration of carbon dioxide dissolved in (meth)acryloyloxyethyl isocyanate was determined in the following manner.

### (1) Preparation of Calibration curve

Toluene was degassed with an aspirator and then standard liquids A, B and C each having a different carbon dioxide concentration each other were prepared in the following methods.
A: Nitrogen was blown for 40 min.
B: Carbon dioxide was blown for 90 sec.
C: Carbon dioxide was blown for 20 min.

In a separating funnel, each of 10 mL of the standard liquids A, B and C was shaken with 10 mL of purified water to extract carbon dioxide. With regard to each liquid, the amount of inorganic carbon in a water phase was measured by a total organic carbon analyzer (hereinafter referred to as a TOC analyzer), and the concentration of carbon dioxide dissolved in the standard liquid was determined in terms of the amount of carbon dioxide dissolved and was taken as a concentration of the standard liquid.

In the meanwhile, each of 1 µL of the standard liquids A, B and C was injected to a gas chromatography mass analyzer (hereinafter referred to as a GC-MS analyzer) to determine an area value of m/z = 44 at a peak of carbon dioxide.

The calibration curve was determined from the concentration of dissolved carbon dioxide determined by the TOC analyzer and the area value determined by the GC/MS analyzer.

### (2) Measurement of Specimen

Into the GC/MS analyzer, 1 µL of the specimen for measurement was injected to determine an area value of m/z = 44 at a peak of the resulting carbon dioxide. From the area value and the calibration curve determined in (1), the amount of carbon dioxide in the specimen was calculated to determine the concentration of carbon dioxide in the specimen.

### Example 1 (Reference only)

To a 2000 mL volume glass reactor equipped with a thermometer, stirrer and hot bath, 1400 g of methacryloyloxyethyl isocyanate having a hydrolyzable chlorine content of 130 ppm (boiling point: 211°C), 224 g of an epoxidized fatty acid ester type plasticizer having an oxirane oxygen content of 6% (molecular weight: about 500, iodine value: 6), 9.8 g of 2,6-di-tert-butyl-4-methyl phenol, 70 g of phenothiazine and 3.78 g of 2-ethyl-4-methyl imidazole were fed and stirred at 120°C for 2 hr. Subsequently, 91 g of phenothiazine was added to the mixture and distilled off at about 0.7 kPa. The initial fraction was collected in an amount of about 10% of the fed amount. Thereafter, a receiving vessel was changed and 900 g of a purified methacryloyloxyethyl isocyanate was prepared. The purified compound had a hydrolyzable chlorine content of 8 ppm.

To the resulting methacryloyloxyethyl isocyanate, a carbon dioxide gas was blown through a glass tube for about 3 min. Subsequently, the concentration of carbon dioxide contained in the methacryloyloxyethyl isocyanate was measured and found to be 243 ppm. Thereafter, when it was kept in a cool and dark condition, a particular change was not observed over 1 month or more. As a result, a stable methacryloyloxyethyl isocyanate was prepared.

### Example 2 (Reference only)

To a 1000 mL volume glass reactor equipped with a thermometer, stirrer and hot bath, 500 g of methacryloyloxyethyl isocyanate having a hydrolyzable chlorine content of 110 ppm (boiling point: 211°C), 80 g of an epoxidized fatty acid ester type plasticizer having an oxirane oxygen content of 6% (molecular weight: about 500, iodine value: 6), 3.5 g of 2,6-di-tert-butyl-4-methyl phenol, 25 g of phenothiazine and 1.35 g of 2-ethyl-4-methyl imidazole were fed and stirred at 120°C for 2 hr. Subsequently, 32.5 g of phenothiazine was added and distilled'off at about 0.7 kPa. The initial fraction was collected in an amount of about 10% of the fed amount. Thereafter, a receiving vessel was changed and 300 g of a purified methacryloyloxyethyl isocyanate was prepared. The purified compound had a hydrolyzable chlorine content of 7 ppm.

To the resulting methacryloyloxyethyl isocyanate, a carbon dioxide gas was blown through a glass tube for about 3 min. Subsequently, the concentration of carbon dioxide contained in the methacryloyloxyethyl isocyanate was measured and found to be 210 ppm. Thereafter, when it was kept in a cool and dark condition, a particular change was not observed over 1 month or more. As a result, a stable methacryloyloxyethyl isocyanate was prepared.

### Example 3 (Reference only)

To a 2000 mL volume glass reactor equipped with a thermometer, stirrer and hot bath, 50 g of methacryloyloxyethyl isocyanate having a hydrolyzable chlorine content of 150 ppm (boiling point: 211°C), 8 g of an epoxidized fatty acid ester type plasticizer having an oxirane oxygen content of 6% (molecular weight: about 500, iodine value: 6), 0.35 g of 2,6-di-tert-butyl-4-methyl phenol and 0.1 g of phenothiazine were fed and stirred at 150°C for 2 hr. Subsequently, distillation thereof was carried out at about 0.7 kPa. The initial fraction was collected in an amount of about 20% of the fed amount. Thereafter, a receiving vessel was changed and 20 g of a purified methacryloyloxyethyl isocyanate was prepared. The purified compound had a hydrolyzable chlorine content of 25 ppm.

To the resulting methacryloyloxyethyl isocyanate, a carbon dioxide gas was blown through a glass tube for about 3 min and degassed by reduced pressure with a vacuum pump for 1 min. Subsequently, the concentration of carbon dioxide contained in the methacryloyloxyethyl isocyanate was measured and found to be 30 ppm. Thereafter, when it was kept in a cool and dark condition, a particular change was not observed over 1 month or more. As a result, a stable methacryloyloxyethyl isocyanate was prepared.

### Example 4

To the resulting methacryloyloxyethyl isocyanate having a hydrolyzable chlorine content of 7 ppm prepared in Example 2 (Reference only), a carbon dioxide gas was blown through a glass tube for about 3 min and degassed by reduced pressure with a vacuum pump for 2 min. Subsequently, the concentration of carbon dioxide contained in the methacryloyloxyethyl isocyanate was measured and found to be 25 ppm. Thereafter, when it was kept in a cool and dark condition, a particular change was not observed over 1 month or more. As a result, a stable methacryloyloxyethyl isocyanate was prepared.

### Comparative Example 1

The procedure of Example 1 (Reference only) was repeated except that a carbon dioxide gas was not blown to the resulting purified methacryloyloxyethyl isocyanate. The concentration of carbon dioxide contained in the methacryloyloxyethyl isocyanate was measured and found to be 13 ppm. Thereafter, it was kept in a cool and dark condition, and then, on the next day, it became whitely turbid.

### Comparative Example 2

The procedure of Example 2 (Reference only) was repeated except that a carbon dioxide gas was not blown to the resulting purified methacryloyloxyethyl isocyanate. The concentration of carbon dioxide contained in the methacryloyloxyethyl isocyanate was measured and found to be 10 ppm. Thereafter, it was kept in a cool and dark condition, and then, on the next day, it became whitely turbid.

### Comparative Example 3

The procedure of Example 3 (Reference only) was repeated except that a carbon dioxide gas was not blown to the resulting purified methacryloyloxyethyl isocyanate. The concentration of carbon dioxide contained in the methacryloyloxyethyl isocyanate was measured and found to be 8 ppm. Thereafter, it was kept in a cool and dark condition, and then, after 3 days, it became whitely turbid.

### Comparative Example 4

The procedure of Example 2 (Reference only) was repeated except that to the resulting purified methacryloyloxyethyl isocyanate, a carbon dioxide gas was blown through a glass tube for about 3 min and thereafter, a nitrogen gas was blown for 10 min to conduct degassing. The concentration of carbon dioxide contained in the methacryloyloxyethyl isocyanate was measured and found to be 15 ppm. Thereafter, it was kept in a cool and dark condition, and then, on the next day, it became whitely turbid.

## Claims

1. A (meth)acryloyloxyalkyl isocyanate having a hydrolyzable chlorine content of not more than 30 ppm based on the (meth)acryloyloxyalkyl isocyanate and containing dissolved carbon dioxide in an amount of not less than 20 ppm based on the (meth)acryloyloxyalkyl isocyanate.

2. The (meth)acryloyloxyalkyl isocyanate according to claim 1 which is prepared by using phosgene.

3. The (meth)acryloyloxyalkyl isocyanate according to claim 1 or 2 wherein the (meth)acryloyloxyalkyl isocyanate is (meth)acryloyloxyethyl isocyanate.

4. A process for stabilizing a (meth)acryloyloxyalkyl isocyanate, which process comprises dissolving carbon dioxide in the (meth)acryloyloxyalkyl isocyanate in an amount of 20 to 250 ppm based on the (meth)acryloyloxyalkyl isocyanate with forced blowing,
wherein the (meth)acryloyloxyalkyl isocyanate is a high purity (meth)acryloyloxyalkyl isocyanate which is prepared by decreasing the amount of hydrolyzable chlorine with purification and has a hydrolyzable chlorine content of not more than 30 ppm based on the (meth)acryloyloxyalkyl isocyanate.

5. The process for stabilizing a (meth)acryloyloxyalkyl isocyanate according to claim 4, wherein the (meth)acryloyloxyalkyl isocyanate is prepared by using phosgene.

6. The process for stabilizing a (meth)acryloyloxyalkyl isocyanate according to claim 4 or 5 wherein the (meth)acryloyloxyalkyl isocyanate is (meth)acryloyloxyethyl isocyanate.

## Patentansprüche

1. (Meth)acryloyloxyalkylisocyanat, das einen Gehalt an hydrolysierbarem Chlor von nicht mehr als 30 ppm, bezogen auf das (Meth)acryloyloxyalkylisocyanat, aufweist und aufgelöstes Kohlendioxid in einer Menge von nicht weniger als 20 ppm, bezogen auf das (Meth)acryloyloxyalkylisocyanat, enthält.

2. (Meth)acryloyloxyalkylisocyanat nach Anspruch 1, hergestellt unter Verwendung von Phosgen.

3. (Meth)acryloyloxyalkylisocyanat nach Anspruch 1 oder 2, wobei das (Meth)acryloyloxyalkylisocyanat (Meth)acryloyloxy-ethylisocyanat ist.

4. Verfahren zum Stabilisieren eines (Meth)acryloyloxyalkyl-isocyanats, wobei das Verfahren das Auflösen von Kohlendioxid in dem (Meth)acryloyloxyalkylisocyanat in einer Menge von 20 bis 250 ppm, bezogen auf das (Meth)acryloyloxyalkylisocyanat, durch Zwangseinblasen umfasst, wobei das (Meth)acryloyloxyalkylisocyanat ein hochreines (Meth)acryloyloxyalkylisocyanat, hergestellt durch Verringern der Menge an hydrolysierbarem Chlor durch Reinigung, ist und einen Gehalt an hydrolysierbarem Chlor von nicht mehr als 30 ppm, bezogen auf das (Meth)acryloyloxyalkylisocyanat, aufweist.

5. Verfahren zum Stabilisieren eines (Meth)acryloyloxyalkylisocyanats nach Anspruch 4, wobei das (Meth)acryloyloxyalkylisocyanat unter Verwendung von Phosgen hergestellt wird.

6. Verfahren zum Stabilisieren eines (Meth)acryloyloxyalkylisocyanats nach Anspruch 4 oder 5, wobei das (Meth)acryloyloxyalkylisocyanat (Meth)acryloyloxyethylisocyanat ist.

## Revendications

1. Isocyanate de (méth)acryloyloxyalkyle présentant une teneur en chlore hydrolysable d'au plus 30 ppm rapporté à l'isocyanate de (méth)acryloyloxyalkyle et contenant du dioxyde de carbone dissous dans une quantité d'au moins 20 ppm rapporté à l'isocyanate de (méth)acryloyloxyalkyle.

2. Isocyanate de (méth)acryloyloxyalkyle selon la revendication 1, lequel est préparé en utilisant du phosgène.

3. Isocyanate de (méth)acryloyloxyalkyle selon la revendication 1 ou 2, dans lequel l'isocyanate de (méth)acryloyloxyalkyle est l'isocyanate de (méth)acryloyloxyéthyle.

4. Procédé de stabilisation d'un isocyanate de (méth)acryloyloxyalkyle, lequel procédé comprend la dissolution de dioxyde de carbone dans l'isocyanate de (méth)acryloyloxyalkyle dans une quantité de 20 à 250 ppm rapporté à l'isocyanate de (méth)acryloyloxyalkyle avec un soufflage forcé,
dans lequel l'isocyanate de (méth)acryloyloxyalkyle est un isocyanate de (méth)acryloyloxyalkyle de pureté élevée qui est préparé par diminution de la quantité de chlore hydrolysable avec purification et présente une teneur en chlore hydrolysable d'au plus 30 ppm rapporté à l'isocyanate de (méth)acryloyloxyalkyle.

5. Procédé de stabilisation d'un isocyanate de (méth)acryloyloxyalkyle selon la revendication 4, dans lequel l'isocyanate de (méth)acryloyloxyalkyle est préparé en utilisant du phosgène.

6. Procédé de stabilisation d'un isocyanate de (méth)acryloyloxyalkyle selon la revendication 4 ou 5, dans lequel l'isocyanate de (méth)acryloyloxyalkyle est l'isocyanate de (méth)acryloyloxyéthyle.
